(19) Europäisches Patentamt
European Patent Office
Office européen des brevets

(11) Veröffentlichungsnummer: **0 241 654 B1**

(12) # EUROPÄISCHE PATENTSCHRIFT

(45) Veröffentlichungstag der Patentschrift: **17.04.91**

(51) Int. Cl.5: **C07D 401/06**, C07D 401/12, A61K 31/44

(21) Anmeldenummer: **87101570.7**

(22) Anmeldetag: **05.02.87**

Die Akte enthält technische Angaben, die nach dem Eingang der Anmeldung eingereicht wurden und die nicht in dieser Patentschrift enthalten sind.

(54) **Hydroxyindolester.**

(30) Priorität: **17.02.86 DE 3604949**

(43) Veröffentlichungstag der Anmeldung:
**21.10.87 Patentblatt 87/43**

(45) Bekanntmachung des Hinweises auf die Patenterteilung:
**17.04.91 Patentblatt 91/16**

(84) Benannte Vertragsstaaten:
**AT BE CH DE ES FR GB IT LI NL SE**

(56) Entgegenhaltungen:
**EP-A- 0 007 399**
**EP-A- 0 105 397**
**EP-A- 0 144 012**
**DE-A- 3 419 935**

(73) Patentinhaber: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **Böttcher, Henning, Dr.**
**Soderstrasse 95**
**W-6100 Darmstadt(DE)**
Erfinder: **Seyfried, Christoph, Dr.**
**Mathildenstrasse 6**
**W-6104 Seeheim-Jugenheim(DE)**
Erfinder: **Minck, Klaus-Otto, Dr.**
**Büchestrasse 5**
**W-6105 Ober-Ramstadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft neue Hydroxyindolester der Formel 1

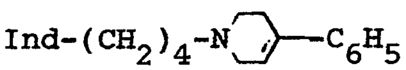

I

worin

Ind

einen in 4-, 5-, 6- oder 7-Stellung durch eine Acyloxygruppe substituierten 3-Indolylrest und

Acyl

Alkanoyl mit 1-4 C-Atomen, Aroyl mit 7-10 C-Atomen, Alkansulfonyl mit 1-4 C-Atomen, Arylsulfonyl mit 6-10 C-Atomen oder Dialkylcarbamoyl mit 3-6 C-Atomen bedeuten, sowie deren Salze.

Der Erfindung lag die Aufgabe zugrunde, neue Verbindungen aufzufinden, die zur Herstellung von Arzneimitteln verwendet werden können.

Es wurde gefunden, daß die Verbindungen der Formel 1 und ihre physiologisch unbedenklichen Salze wertvolle pharmakologische Eigenschaften besitzen. So zeigen sie insbesondere Wirkungen auf das Zentralnervensystem, vor allem dopaminstimulierende präsynaptische (neuroleptische) oder postsynaptische (Anti-Parkinson) Wirkungen. Im einzelnen induzieren die Verbindungen der Formel 1 contralaterales Drehverhalten in Hemiparkinson-Ratten (feststellbar nach der Methode von Ungerstedt et al., Brain Res. 24 (1970), 485-493) und hemmen die Bindung von tritiierten Dopaminagonisten und -antagonisten an striäre Rezeptoren (feststellbar nach der Methode von Schwarcz et al., J. Neurochemistry 34 (1980), 772-778, und Creese et al., European J. Pharmacol. 46 (1977), 377-381). Zusätzlich hemmen die Verbindungen den Zungen-Kieferreflex bei der narkotisierten Ratte (feststellbar in Anlehnung an die Methoden von Barnett et al., European J. Pharmacol. 21 (1973), 178-182, und von Ilhan et al., European J. Pharmacol. 33 (1975), 61-64). Weiterhin treten analgetische und blutdrucksenkende Wirkungen auf; so wird der bei kathetertragenden wachen, spontan hypertonen Ratten (Stamm SHR/NIH-MO/CHB-EMD; Methode vgl. Weeks und Jones, Proc. Soc. Exptl. Biol. Med. 104 (1960), 646-648) direkt gemessene Blutdruck nach intragastraler Gabe der Verbindungen gesenkt.

Verbindungen der Formel 1 und ihre physiologisch unbedenklichen Salze können daher als Arzneimittelwirkstoffe und auch als Zwischenprodukte zur Herstellung anderer Arzneimittelwirkstoffe verwendet werden.

Gegenstand der Erfindung sind die Indolderivate der Formel 1 sowie ihre Salze.

In der Acyloxygruppe, durch die der Rest Ind substituiert ist, bedeutet "Acyl" Alkanoyl mit 1-4 C-Atomen wie Formyl, Acetyl, Propionyl, Butyryl, Isobutyryl; Aroyl mit 7-10 C-Atomen wie Benzoyl, o-, m- oder p-Toluyl, o-, m- oder p-Ethylbenzoyl, 1- oder 2-Naphthoyl; Alkansulfonyl mit 1-4 C-Atomen wie Methansulfonyl, Ethansulfonyl, 1- oder 2-Propansulfonyl; Arylsulfonyl mit 6-10 C-Atomen wie Benzolsulfonyl, o-, m-, oder p-Toluolsulfonyl, 1- oder 2-Naphthalinsulfonyl.

Dialkylcarbamoyl mit 3-6 C-Atomen wie N,N-Dimethylcarbamoyl, N,N-Diethylcarbamoyl.

Gegenstand der Erfindung ist ferner ein Verfahren zur Herstellung der Verbindungen der Formel 1 sowie ihrer Salze, dadurch gekennzeichnet, daß man eine Verbindung der Formel II

$$Ind\text{-}(CH_2)_4\text{-}X^1 \qquad II$$

worin

$X^1$

X oder $NH_2$ und

X

Cl, Br, J, 0H oder eine reaktionsfähig funktionell abgewandelte 0H-Gruppe bedeuten und

Ind

die angegebene Bedeutung hat,

mit einer Verbindung der Formel III

$$X^2\text{-}CH_2CH_2\text{-}C(C_6H_5)\text{=}CHCH_2\text{-}X^3 \qquad III$$

worin
$X^2$ und $X^3$
gleich oder verschieden sein können und, falls $X^1$ = $NH_2$ ist, jeweils X, andernfalls zusammen NH bedeuten und
X
die angegebene Bedeutung hat,
umsetzt
oder daß man eine sonst der Formel I entsprechende Verbindung, die jedoch an Stelle eines oder mehrerer Wasserstoffatome eine oder mehrere reduzierbare Gruppe(n) und/ oder eine oder mehrere zusätzliche C-C- und/oder C-N-Bindung(en) enthält, mit einem reduzierenden Mittel behandelt.

Die Herstellung der Verbindungen der Formel I erfolgt im übrigen nach an sich bekannten Methoden, wie sie in der Literatur (z. B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel 1 umsetzt.

Vorzugsweise sind die Hydroxyindolester der Formel 1 durch Veresterung der entsprechenden Hydroxyindole erhältlich. Diese sind teilweise bekannt, z. B. aus der EP-A-0007399 oder der EP-A-0105397; die nicht bekannten können leicht in Analogie zu den bekannten hergestellt werden, z. B. durch Reaktion von Hydroxyindolderivaten entsprechend der Formel Ind-$(CH_2)_4$-$X^1$, worin aber an Stelle des Restes Ind ein in 4-, 5-, 6- oder 7-Stellung durch eine HO-Gruppe substituierter 3-Indolylrest steht, mit Verbindungen der Formel 111.

Die Veresterung wird zweckmäßig mit Hilfe eines reaktiven Derivats der entsprechenden Säure durchgeführt, z. B. eines Anhydrids, Chlorids oder Bromids. Man arbeitet in Gegenwart oder Abwesenheit eines inerten Lösungsmittels, z. B. eines Kohlenwasserstoffs wie Benzol oder Dichlormethan zweckmäßig unter Zusatz einer Base wie Natron- oder Kalilauge oder eines tertiären Amins wie Triethylamin, Pyridin oder 4-Dimethylaminopyridin. Ein Überschuß der Base kann auch als Lösungsmittel dienen. Die Reaktionstemperaturen liegen vorzugsweise zwischen 0 und 150°, insbesondere zwischen 20 und 120°.

In den Indolderivaten der Formel II ist $X^1$ vorzugsweise x, dementsprechend sind in den Verbindungen der Formel 111 $X^2$ und $x^3$ vorzugsweise zusammen NH. Der Rest X ist vorzugsweise Cl oder Br, er kann jedoch auch J, OH oder eine reaktionsfähig funktionell abgewandelte 0H-Gruppe bedeuten, insbesondere Alkylsulfonyloxy mit 1-6 (z. B. Methansulfonyloxy) oder Arylsulfonyloxy mit 6-10 C-Atomen (z. B. Benzolsulfonyloxy, p-Toluolsulfonyloxy, 1- oder 2-Naphthalin-sulfonyloxy).

Dementsprechend sind die Indolderivate der Formel 1 insbesondere durch Umsetzung von Verbindungen der Formel Ind-$(CH_2)_4$-Cl oder Ind-$(CH_2)_4$-Br mit der Verbindung der Formel 111, worin $X^2$ und $X^3$ zusammen eine NH-Gruppe bedeuten (nachstehend als 111a bezeichnet) erhältlich.

Die Verbindungen der Formeln 11 und 111 sind zum Teil bekannt; die nicht bekannten Verbindungen der Formeln 11 und 111 können leicht analog zu den bekannten Verbindungen hergestellt werden. Primäre Alkohole der Formel Ind-$(CH_2)_4$-OH sind z. B. durch Veresterung der entsprechenden Carbonsäuren und anschließende selektive Reduktion oder durch selektive Veresterung erhältlich. Behandeln mit Thionylchlorid, Bromwasserstoff, Phosphortribromid oder ähnlichen Halogenverbindungen liefert die entsprechenden Halogenide der Formel Ind-$(CH_2)_4$-Hal. Die entsprechenden Sulfonyloxyverbindungen sind erhältlich aus den Alkoholen Ind-$(CH_2)_4$-OH durch Umsetzung mit den entsprechenden Sulfonsäurechloriden. Die Jodverbindungen der Formel Ind-$(CH_2)_4$-J sind z. B. durch Einwirkung von Kaliumjodid auf die zugehörigen p-Toluolsulfonsäureester erhältlich. Die Amine der Formel Ind-$(CH_2)_4$-$NH_2$ sind z. B. aus den Halogeniden mit Phthalimidkalium oder durch Reduktion der entsprechenden Nitrile herstellbar.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z. B. Kohlenwasserstoffe, wie Benzol, Toluol, Xylol; Ketone wie Aceton, Butanon; Alkohole wie

Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali-oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente Ind-$(CH_2)_4$-$NH_2$ bzw. IIIa kann begünstigt sein. Die Reaktionszeit liegt je nach den angewendeten Bedingungen zwischen einigen Minuten und 14 Tagen, die Reaktionstemperaturen zwischen etwa 0 und 150°, normalerweise zwischen 20 und 130°.

Es ist ferner möglich, eine Verbindung der Formel I zu erhalten, indem man ein Vorprodukt, das an Stelle von Wasserstoffatomen eine oder mehrere reduzierbare Gruppe(n) und/oder eine oder mehrere zusätzliche C-C-und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z. B. p-Toluolsulfonyloxy), N-Benzol-sulfonyl, N-Benzyl oder O-Benzyl.

So gelingt z. B. eine Reduktion von Pyridiniumsalzen der Formel IV

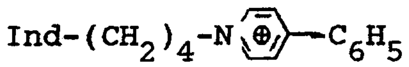 $An^{\ominus}$ IV

worin An ein Anion, vorzugsweise Cl, Br oder $CH_3SO_3$ bedeutet, zu Verbindungen der Formel 1 z. B. mit $NaBH_4$ in Wasser, Methanol oder Ethanol oder in Gemischen dieser Lösungsmittel, falls erwünscht unter Zusatz einer Base wie NaOH, bei Temperaturen zwischen etwa 0 und 80°.

Eine erhaltene Base der Formel I kann mit einer Säure in das zugehörige Säureadditionssalz überführt werden. Für diese Umsetzung eignen sich Säuren, die physiologisch unbedenkliche Salze liefern. So können anorganische Säuren verwendet werden, z. B. Schwefelsäure, Halogenwasserstoffsäuren wie Chlorwasserstoffsäure oder Bromwasserstoffsäure. Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Oernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalinmono- oder -disulfonsäuren, Laurylschwefelsäure. Salze mit physiologisch nicht unbedenklichen Säuren, z. B. Pikrate, können zur Isolierung oder Aufreinigung der Verbindungen der Formel I verwendet werden.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Die Verbindungen der Formel I und ihre physiologisch unbedenklichen Salze können zur Herstellung pharmazeutischer Zubereitungen verwendet werden, imsbesondere auf nichtchemischen Wege. Hierbei können sie zusammen mit mindestens einem festen, flüssigen oder halb-flüssigen Träger- oder Hilfsstoff und gegebenenfalls in Kombination mit einer oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- oder Veterinärmedizin eingesetzt werden. Als Trägersubstanzen kommen organische oder anorganische Stoffe in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs-und/oder Netzmittel, Emulgatoren, Salze zur Beeinflussung des osmotischen Druckes, Puffersubstanzen, Farb-, Geschmacks- und/oder Aromastoffe enthalten. Sie können, falls

erwünscht, auch einen oder mehrere weitere Wirkstoffe enthalten, z. B. ein oder mehrere Vitamine.

Die Verbindungen der Formel 1 und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers und bei der Bekämpfung von Krankheiten verwendet werden, insbesondere bei der Therapie von Parkinsonismus, von extrapyramidalen Störungen bei der Neuroleptikatherapie, von Depressionen und/oder Psychosen und von Nebenwirkungen bei der Behandlung der Hypertonie (z. B. mit α-Methyl-dopa). Ferner können die Verbindungen in der Endokrinologie und Gynäkologie Verwendung finden, z. B. zur Therapie von Akromegalie, Hypogonadismus, sekundärer Amenorrhoe, prämenstruellem Syndrom, unerwünschter puerperaler Laktation und generell als Prolaktin-Hemmer, weiterhin zur Therapie cerebraler Störungen (z. B. Migräne), insbesondere in der Geriatrie ähnlich wie gewisse Ergot-Alkaloide, sowie auch zur Blutdrucksenkung.

Dabei werden die erfindungsgemäßen Substanzen in der Regel in Analogie zu bekannten, im Handel befindlichen Präparaten (z. B. Bromocriptin, Dihydroergocomin) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden bestimmten Patienten hängt jedoch von dem verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/ oder durch Kristallisation. Temperaturen sind in °C angegeben. RE-Werte wurden dünnschichtchromatographisch an Kieselgel ermittelt.

Beispiel 1

Zu einer Lösung von 34,6 g 3-[4-(4-Phenyl-3,4-dehydro-1-piperidyl)-butyl]-5-hydroxyinol in 175 ml Pyridin tropft man 10,5 ml Acetanhydrid, erhitzt 30 Min. auf dem Dampfbad, gießt in 1,7 l Wasser, rührt noch 1 Std. und filtriert das erhaltene 3-[4-(4-Phenyl-3,4-dehydro-1-piperidyl )-butyl]-5-acetoxy-indol ("A") ab. F. 120-122° (aus Isopropanol).

Analog erhält man mit den Anhydriden, Chloriden oder Bromiden der entsprechenden Säuren die nachstehenden 3-[4-(4-Phenyl-3 ,4-dehydro-l-piperidyl )-butyl-indole:

4-Acetoxy-
6-Acetoxy-
7-Acetoxy-
5-Propionyloxy-
5-Butyryloxy-
5-Isobutyryloxy-
5-Benzoyloxy-, flydrochlorid-hydrat, F. 106° (Zers.)
5-p-Toluyloxy-
4-Methansulfonyloxy-
5-Methansulfonyloxy-, Hydrochlorid, F. 208-210°
6-Methansulfonyloxy-
7-Methansulfonyloxy-
5-Ethansulfonyloxy-
4-Benzoylsulfonyloxy-
5-Benzoylsulfonyloxy-
6-Benzoylsulfonyloxy-
7-Benzoylsulfonyloxy-
4-p-Toluolsulfonyloxy-
5-p-Toluolsulfonyloxy-, Hydrochlorid, F. 226-228°
6-p-Toluolsulfonyloxy-
7-p-Toluolsulfonyloxy-

5-N,N-Dimethylcarbamoyloxy-, Hydrochlorid, F. 180-182°
5-N,N-Diethylcarbamoyloxy-

Beispiel 2

Man rührt eine Lösung von 2,66 g 3-(4-Chlorbutyl)-5-acetoxy-indol (oder 3,10 g 3-(4-Brombutyl)-5-acetoxy-indol) und 1,6 g 4-Phenyl-3,4-dehydropiperidin (IIIa) in 10 ml Acetonitril 12 Std. bei 20°, arbeitet wie üblich auf und erhält "A", F. 120-122°.

Beispiel 3

Ein Gemisch von 2,46 g 3-(4-Aminobutyl)-5-acetoxy-indol [erhältlich durch Reaktion von 3-(4-Brombutyl)5-acetoxy-indol mit Phthalimidkalium und anschließende Hydrolyse] und 2,15 g 1,5-Dichlor-3-phenyl-2penten in 40 ml Aceton und 40 ml Wasser wird 24 Std. gekocht und wie üblich aufgearbeitet. Man erhält "A", F. 120-122°.

Beispiel 4

Zu einer Lösung von 46,5 g (1-[4-(5-Acetoxy-3-indolyl)-butyl]-4-phenylpyridiniumbromid [erhältlich aus 4-Phenyl-pyridin und 3-(4-Brombutyl)-5-acetoxy-indol] in 500 ml wässeriger 1 n NaOH gibt man unter Rühren 10 g $NaBH_4$ in 200 ml Wasser und rührt danach noch 3 Std. bei 60°. Nach üblicher Aufarbeitung erhält man "A", F. 120-122°.

Beispiel A: Tabletten

Ein Gemisch von 1 kg 3-[4-(4-Phenyl-3,4-dehydro-1-pipe-ridyl)-butyl]-5-acetoxy-indol, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 10 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

2 kg 3-[4-(4-Phenyl-3,4-dehydro-1-piperidyl)buty1]-5-acetoxy-indol werden in üblicher Weise in Hartgelatinekapseln gefüllt, so daß jede Kapsel 20 mg des Wirkstoffs enthält.

Beispiel D: Ampullen

Eine Lösung von 1 kg 3-[4-(4-Phenyl-3,4-dehydro-1-pipe-ridyl)-butyl]-5-acetoxy-indol in 30 1 zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel 1 und/oder ihre physiologisch unbedenklichen Säureadditionssalze enthalten.

Im Spiperon-Bindungs-Test an Ratten nach Creese et al. (1.c.) waren die folgenden 3-[4-(4-Phenyl-3,4-dehydro-1-piperidyl)-butyl]-indole besonders wirksam ([$^3$H]-Spiperon-Bindung bei $10^{-7}$ mol . $1^{-1}$ geringer als 25 %, bezogen auf Kontrollwerte = 100 %):
5-p-Toluolsulfonyloxy-
5-Methansulfonyloxy-

5-Acetoxy-
5-N,N-Dimethylcarbamoyloxy-.

## Ansprüche

**1.** Hydroxyindolester der Formel I

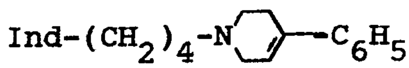 I

worin
Ind
einen in 4-, 5-, 6- oder 7-Stellung durch eine Acyloxygruppe substituierten 3-Indolylrest
und
Acyl
Alkanoyl mit 1-4 C-Atomen, Aroyl mit 7-10 C-Atomen, Alkansulfonyl mit 1-4 C-Atomen, Arylsulfonyl mit 6-10 C-Atomen oder Dialkylcarbamoyl mit 3-6 C-Atomen
bedeuten,
sowie deren Salze.

**2.**

a) 3-[4-(4-Phenyl-3,4-dehydro-l-piperidyl)-butyl]-5-acetoxy-indol;
b) 3-[4-(4-Phenyl-3,4-dehydro-1-piperidyl)-butyl]-5-methansulfonyloxy-indol.

**3.** Verfahren zur Herstellung von Verbindungen der Formel I sowie von deren Salzen, dadurch gekennzeichnet, daß man ein entsprechendes Hydroxyindol verestert, oder daß man eine Verbindung der Formel II

$$Ind\text{-}(CH_2)_4\text{-}X^1 \qquad II$$

worin
$X^1$
X oder $NH_2$ und
X
Cl, Br, J, 0H oder eine reaktionsfähig funktionell abgewandelte 0H-Gruppe bedeuten und
Ind
die angegebene Bedeutung hat,
mit einer Verbindung der Formel III

$$X^2\text{-}CH_2CH_2\text{-}C(C_6H_5)\text{=}CHCH_2\text{-}X^3 \qquad III$$

worin
$X^2$ und $X^3$
gleich oder verschieden sein können und, falls $x^1$ = $NH_2$ ist, jeweils X, andernfalls zusammen NH bedeuten und
X
die angegebene Bedeutung hat,
umsetzt

oder daß man eine sonst der Formel I entsprechende Verbindung, die jedoch an Stelle eines oder mehrerer Wasserstoffatome eine oder mehrere reduzierbare Gruppe(n) und/oder eine oder mehrere zusätzliche c-c-und/oder C-N-Bindung(en) enthält, mit einem reduzierenden Mittel behandelt und/oder daß man eine erhaltene Base der Formel I durch Behandeln mit einer Säure in eines ihrer Säureadditionssalze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoffen in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel I oder ihre physiologisch unbedenklichen Salze zur Bekämpfung von Krankheiten.

7. Verwendung von Verbindungen der Formel I oder ihren physiologisch unbedenklichen Salzen zur Herstellung von Arzneimitteln.

## Claims

1. Hydroryindole esters of the formula I

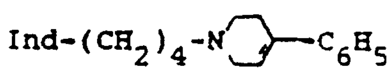

I

wherein
Ind is a 3-indolyl radical which is substituted in the 4-, 5-, 6- or 7-position by an acyloxy group and
Acyl is alkanoyl having 1-4 C atoms, aroyl having 7-10 C atoms, alkanesulfonyl having 1-4 C atoms, arylsulfonyl having 6-10 C atoms or dialkylcarbamoyl having 3-6 C atoms, and salts thereof.

2.

(a) 3-[4(4-Pheny1-3,4-dehydro-1-piperidyl)-butyl ]-5-acetoryindole;
(b) 3-[4-(4-Pheny1-3,4-dehydro-1-piperidyl)-butyl]-5-methanesulfonyloxyindole.

3. Process for the preparation of compounds of the formula I and salts thereof, characterised in that a corresponding hydroxyindole is esterified, or a compound of the formula II

$$Ind-(CH_2)_4-X^1 \qquad II$$

wherein
$X^1$ is X or $NH_2$ and
X is Cl, Rr, I, OH or a reactive, functionally modified OH group and
Ind has the meaning indicated,
is reacted with a compound of the formula III

$$X^2-CH_2CH_2-C(C_6H_5)=CHCH_2-X^3 \qquad III$$

wherein $X^2$ and $X^3$ can be identical or different and, if
$X^1$ is $NH_2$, are each X and otherwise together denote NH and

X has the meaning indicated,
or
a compound which otherwise corresponds to the formula I, but which contains one or more reducible -group(s) and/or one or more additional C-C-and/or C-N- bond(s) instead of one or more hydrogen atoms is treated with a reducing agent and/or a resulting base of the formula I is converted into one of its acid addition salts by treatment with an acid.

**4.** Process for the preparation of pharmaceutical formulations, characterised in that a compound of the formula I and/or one of its physiologically acceptable salts is brought into a suitable dosage form together with at least one solid, liquid or semi-liquid excipient or auxiliary and, if desired, in combination with one or more further active compounds.

**5.** Pharmaceutical formulation characterised in that it contains at least one compound of the formula I and/or one of its physiologically acceptable salts.

**6.** Compounds of the formula I or physiologically acceptable salts thereof for combating diseases.

**7.** The use of compounds of the formula I or physiologically acceptable salts thereof for the preparation of medicaments.

## Revendications

**1.** Esters d'hydroxyindole de formule I

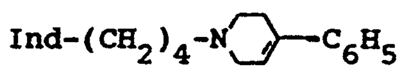

I

où
Ind
représente un reste 3-indolyle substitué sur la position 4, 5, 6 ou 7 par un groupe acyloxy et
Acyl
représente les groupes alcanoyle en $C_1$-$C_4$, aroyle en $C_7$-$C_{10}$, alcane-sulfonyle en $C_1$-$C_4$, arylsulfonyle en $C_6$-$C_{10}$ ou dialkylcarbamoyle en $C_3$-$C_6$,
ainsi que leurs sels.

**2.**

a) 3-[4-(4-phényl-3,4-déhydro-1-pipéridyl)-butyl]-5-acétoxy-indole ;
b) 3-[4-(4-phényl-3,4-déhydro-1-pipéridyl)-butyl]-5-méthane-sulfonyloxy-indole.

**3.** Procédé pour la préparation de composés de formule I, ainsi que de leurs sels, caractérisé en ce qu'on estérifie un hydroxyindole correspondant, ou que l'on fait réagir un composé de formule II

$$\text{Ind-}(CH_2)_4\text{-}X^1 \qquad \text{II}$$

où
$X^1$
est X ou $NH_2$ et
X
représente C1, Br, I, 0H ou un groupe 0H fonctionnellement modifié réactif, et
Ind
a la signification susdite,
avec un composé de formule III

$$X^2-CH_2CH_2-C(C_6H_5)=CHCH_2-X^3 \qquad III$$

où

$X^2$ et $X^3$

peuvent être identiques ou différents et, dans le cas où $x^1 = NH_2$, sont chacun X, sinon forment ensemble NH, et

X

a la signification susdite,

ou que l'on traite avec un agent réducteur un composé correspondant par ailleurs à la formule I, mais comportant à la place d'un ou plusieurs atomes d'hydrogène un ou plusieurs groupe(s) réductible(s) et/ou une ou plusieurs liaison(s) supplémentaire(s) C-C et/ou C-N, et/ou que l'on transforme une base obtenue répondant à la formule I par traitement avec un acide en un de ses sels d'addition à un acide.

**4.** Procédé pour l'obtention de préparations pharmaceutiques, caractérisé en ce qu'on met sous une forme de dosage appropriée un composé de formule I et/ou un de ses sels physiologiquement acceptables conjointement avec au moins un support ou excipient solide, liquide ou semi-fluide et éventuellement en combinaison avec un ou plusieurs autre(s) principe(s) actif(s).

**5.** Préparation pharmaceutique, caractérisée en ce qu' elle comporte au moins un composé de formule I et/ou un de ses sels physiologiguement acceptables.

**6.** Composés de formule I ou leurs sels physiologiquement acceptables pour la lutte contre les maladies.

**7.** Utilisation des composés de formule I ou de leurs sels physiologiquement acceptables pour la préparation de médicaments.